# FASCICULE DE BREVET EUROPEEN

(11) **EP 1 635 804 B1**
(45) Date de publication et mention de la délivrance du brevet: **21.09.2016**
(21) Numéro de dépôt: 04767222.5
(22) Date de dépôt: 01.06.2004
(51) Int. Cl.: A61K 31/137, A61K 31/138, A61K 31/165, A61K 31/198, A61K 31/295, A61K 31/4458, A61K 33/26, A61K 45/06, A61P 25/00

(54) **UTILISATION DU FER POUR LE TRAITEMENT DU TROUBLE DU DEFICIT DE ATTENTION/HYPER-ACTIVITE CHEZ LES ENFANTS**
VERWENDUNG VON EISEN ZUR BEHANDLUNG VON AUFMERKSAMKEITSDEFIZITEN MIT HYPERAKTIVITÄT BEI KINDERN
USE OF IRON FOR TREATING ATTENTION DEFICIT HYPERACTIVITY DISORDER IN CHILDREN

(30) Priorité: 30.05.2003 FR 0306581
(43) Date de publication de la demande: 22.03.2006
(60) Demande divisionnaire: 10195688.6
(73) Titulaire: NLS-1 Pharma AG, 6370 Stans (CH)
(72) Inventeur: KONOFAL, Eric, F-75015 Paris (FR)
(74) Mandataire: Regimbeau
(86) Numéro de dépôt international: PCT/FR2004/001351
(87) Numéro de publication internationale: WO 2004/105744

(56) Documents cités:
- SEVER YONATHAN ET AL: "Iron treatment in children with attention deficit hyperactivity disorder. A preliminary report" NEUROPSYCHOBIOLOGY, vol. 35, no. 4, 1997, pages 178-180, XP009024790 ISSN: 0302-282X cité dans la demande
- DAVIS B J ET AL: "A RANDOMIZED, DOUBLE-BLIND PLACEBO-CONTROLLED TRIAL OF IRON IN RESTLESS LEGS SYNDROME" EUROPEAN NEUROLOGY, XX, XX, vol. 43, no. 2, 2000, pages 70-75, XP008019659
- BURATTINI M G ET AL: "[Evaluation of the effectiveness of gastro-protected proteoferrin in the therapy of sideropenic anemia in childhood]" MINERVA PEDIATRICA. ITALY SEP 1990, vol. 42, no. 9, septembre 1990 (1990-09), pages 343-347, XP009024864 ISSN: 0026-4946
- O'KEEFFE S T ET AL: "Iron status and restless legs syndrome in the elderly." AGE AND AGEING. ENGLAND MAY 1994, vol. 23, no. 3, mai 1994 (1994-05), pages 200-203, XP009024887 ISSN: 0002-0729
- SUN ERICA R ET AL: "Iron and the restless legs syndrome" SLEEP (ROCHESTER), vol. 21, no. 4, 15 juin 1998 (1998-06-15), pages 371-377, XP009024868 ISSN: 0161-8105
- SCHAUSS A G: "Nutrition and behavior: complex interdisciplinary research." NUTRITION AND HEALTH (BERKHAMSTED, HERTFORDSHIRE) ENGLAND 1984, vol. 3, no. 1-2, 1984, pages 9-37, XP009024863 ISSN: 0260-1060
- WARD NEIL I: "Assessment of chemical factors in relation to child hyperactivity" JOURNAL OF NUTRITIONAL AND ENVIRONMENTAL MEDICINE (ABINGDON), vol. 7, no. 4, décembre 1997 (1997-12), pages 333-342, XP009024867 ISSN: 1359-0847
- WALTHER BJÖRN WITO: "Treating restless legs syndrome: current pathophysiological concepts and clinical trials." EXPERT OPINION ON INVESTIGATIONAL DRUGS. ENGLAND APR 2002, vol. 11, no. 4, avril 2002 (2002-04), pages 501-514, XP009024933 ISSN: 1354-3784
- KONOFAL ET AL: "Effects of Iron Supplementation on Attention Deficit Hyperactivity Disorder in Children", PEDIATRIC NEUROLOGY, ELSEVIER SCIENCE, NL, vol. 38, no. 1, 3 December 2007 (2007-12-03), pages 20-26, XP022370910, ISSN: 0887-8994, DOI: 10.1016/J.PEDIATRNEUROL.2007.08.014

## Description

La présente invention concerne le domaine de la santé humaine et plus particulièrement le traitement du trouble "déficit de l'attention/hyper-activité". Plus particulièrement, la présente invention concerne l'utilisation du fer ou l'un de ses sels pharmaceutiquement acceptables, seul ou en association avec un ou plusieurs composés psycho-stimulants, pour la préparation d'un médicament destiné au traitement de la TDAH.

Le trouble "déficit de l'attention/hyper-activité" de l'enfant (TDAH) est un trouble comportemental qui constitue le premier motif de consultation en psychologie de l'enfant et de l'adolescent. Ce syndrome très répandu affecte 6 à 10 % des enfants d'âge scolaire.

Sur le plan clinique, ce trouble associe une inattention, une impulsivité et une hyperactivité motrice inadaptée à l'environnement de l'enfant. Mal organisés et étourdis, ces enfants finissent parfois par ne plus suivre en classe. L'agitation motrice excessive, incompatible avec les relations sociales et pouvant parfois même conduire à une déscolarisation prématurée, est probablement le symptôme qui amènera les parents à consulter un spécialiste.

La physiopathologie de ce trouble reste encore aujourd'hui discutée bien que, pour un bon nombre d'auteurs, l'hypothèse d'une implication des systèmes dopaminergiques et noradrénergiques semble validée [Spencer et al., Pharmacotherapy of attention déficit hyperactivity disorder. Child Adolesc Psychiatr Clin N Am. 2000; 9(1):77-97]. Ce dysfonctionnement de la neurotransmission dopaminergique semble être impliqué dans les symptômes d'hyperactivité motrice excessive caractéristique du TDAH de l'enfant. De fait, l'amélioration de l'hyperactivité motrice par les psychostimulants dopaminergiques est souvent très significative mais néanmoins insuffisante.

Les insomnies, les difficultés d'endormissement, les réveils au cours de la nuit, éventuellement dus à une agitation motrice nocturne excessive, ainsi que les troubles attentionnels, tels que l'inattention, l'impatience et l'impulsivité semblent échapper à toute forme de traitement [Chervin et al., Associations between symptoms of inattention, hyperactivity, restless legs, and periodic leg movements. Sleep 2002 15;25(2):213-8; Gruber et al., instability of sleep patterns in children with attention-deficit/hyperactivity disorder. J Am Acad Child Adolesc Psychiatry. 2000;39(4):495-501].

Il existe donc un réel besoin de développer de nouveaux traitements du TDAH qui permettent d'obtenir des résultats supérieurs à ceux obtenus avec les traitements actuels à base de psycho-stimulants et notamment de pouvoir traiter les symptômes qui échappent aux traitements actuels. C'est le but de la présente invention.

De manière tout à fait fortuite, l'inventeur a maintenant observé une ferritinémie anormalement basse chez les enfants atteints de TDAH. L'inventeur a en outre démontré une corrélation entre la sévérité des symptômes et la férritinémie. La présente invention se propose donc de fournir un traitement préventif et curatif du TDAH par la correction de l'hypoferritinémie observée chez ces patients.

Par le passé, différentes études ont été menées qui ont conduit à étudier l'implication du fer dans le TDAH. A l'origine de ces études est la constatation que des enfants atteints de TDAH ont un concentration basse en certains oligoéléments, dont le fer (Pour revue voir : Brue and Oakland, 2002 Alternatives Thérapies 8 :68-73). Ainsi, en 1994, Kozielec et al. (Psychiatr.pol. 28 : 345-353) ont recherché un déficit en oligoéléments (magnésium, zinc, cuivre, calcium, fer) dans le TDAH. Les auteurs n'ont pas fait de lien entre le fer et le TDAH, et à l'inverse, la magnésémie retrouvée chez les enfants étudiés suggère aux auteurs d'évoquer l'intérêt d'une supplémentation en magnésium dans le TDAH. L'existence d'une motricité nocturne excessive a conduit Sever et al. (1997, Neuropsychobiology 35 :178-180) à étudier l'implication du fer dans le TDAH d'enfants non anémiés. Cette étude, qui reste la seule étude s'étant intéressée à montrer l'intérêt de traiter empiriquement des enfants présentant un TDAH, n'évoque pas dans ses résultats ni dans ses perspectives le rôle de la ferritine dans la physiopathologie du trouble. Les résultats obtenus ne montrent pas de rapport entre la sévérité des symptômes et la carence martiale. Les auteurs ont d'ailleurs conclu que le déficit en fer ne joue pas un rôle dans la physiopathologie du TDAH et qu'un traitement oral par le fer des enfants affectés de TDAH n'est pas recommandé. Davis et al. (European Neurology 2000, 43, 70-75) décrit une étude clinique randomisée en double aveugle destinée à évaluer l'effet d'une supplémentation en fer (325 mg/jour de sulfate de fer) sur des patients non anémiés présentant le syndrome des jambes sans repos, dont certains prennent un traitement à base de levoDOPA. Il n'est cependant pas indiqué si ces patients présentent une carence martiale. Les auteurs concluent à l'absence d'effet d'une telle supplémentation en fer sur le syndrome des jambes sans repos.
Burattini et al (Minerva Pediatrica, 1990, 42, 9, 343-347) décrit une étude réalisée chez des enfants de 6 mois à 15 ans atteints d'une carence en fer et/ou d'anémie sidéropénique traités par de la ferritine pendant 4 mois à raison de 4 à 5 mg/kg/jour. Ce traitement a permis d'améliorer les paramètres sanguins : hémoglobine, ferritine, et les symptômes associés à l'anémie : pâleur, anorexie, débilité, somnolence, hyperactivité, sommeil perturbé et sudation excessive. Burattini et al ne décrit pas l'utilisation du fer pour la préparation d'un médicament destiné au traitement du TDAH, Trouble du Déficit de l'Attention et de l'Hyperactivité, qui est un syndrome idiopathique en tant que tel décrit dans le DSM IV non lié à l'anémie.
Les documents O'Keefe et al (Age and Ageing, 1994, 23, 3, 200-203), Sun et al (Sleep (Rochester) 1998, 21, 4, 371-377) et Walther (Expert Opinion on Investigational Drugs, 2002, 11, 4, 501-514) divulguent les résultats d'études cliniques menées sur des sujets présentant le syndrome des jambes sans repos. Les patients ont tous reçus une supplémentation en fer, mais les conclusions des articles quant aux effets d'un tel traitement sur les symptômes divergent. De plus, aucun de ces articles ne divulgue un traitement combiné fer/psychostimulant. Le document Schauss et al (Nutrition and Health 1984, 3, 1-2, 9-37)) est une revue consacrée à l'influence de la nourriture et des nutriments sur divers aspects comportementaux. Cependant, cet article associe une carence en fer uniquement à des comportements violents. Enfin, l'article de Ward (Journal of Nutritional and Environmental Medicine, 1997, 7, 4, 333-342) concerne l'influence de l'ingestion de colorants alimentaires sur les symptômes du TDAH chez des enfants et mentionne une éventuelle corrélation entre le taux sanguin de fer et de zinc et l'hyperactivité. Cependant, cet article se conceritre surtout sur les colorants alimentaires, et semble conclure à l'influence prépondérante du zinc. En aucun cas cet article ne suggère une thérapie concernant le TDAH. Finalement, ces résultats inconsistants et parfois contradictoires obtenus par les chercheurs et les médecins les ont conduit à privilégier d'autres approches thérapeutiques du TDAH qui ont cours actuellement, à base notamment de psycho-stimulants dopaminergiques.

La présente invention a donc pour objet l'utilisation du fer, ou l'un de ses sels pharmaceutiquement acceptables, pour la préparation d'un médicament destiné au traitement préventif et/ou curatif du trouble du déficit de l'attention/hyperactivité (TDAH). Par une posologie martiale correspondant a une prise journaliere de sulfate ferreux comprise entre 100 mg et 2 g par jour, de preference environ 500 mg, en une ou plusieurs prises chez un patient nécessitant un tel traitement. La ferritine est une protéine de stockage du fer (Connor et al., Pediatric Neurology 25 : p123-124).

Dans le cadre de la présente invention, le diagnostic de Trouble Déficit de l'Attention/Hyperactivité (TDAH) est fondé selon les caractéristiques cliniques définies par la classification internationale, le Manuel Diagnostique et Statistique des troubles mentaux, DSM-IV (Diagnostic and Statistical Manual of mental disorders, 4ème ed., 1994).

Les critères du DSM-IV incluent trois dimensions (inattention, impulsivité et hyperactivité), une efficience intellectuelle normale (QI>80), mais n'incluent aucune pathologie organique, ni neurologique.

Dans le cas de la présente invention, le patient est donc un enfant avec un QI>80, d'âge compris entre 5 et 12 ans, et présentant une carence martiale isolée mais non anémié, c'est-à-dire présentant un taux d'hémoglobine normal. Par l'expression "carence martiale", on entend une hypoferritinémie sans modification significative de la concentration sérique en récepteurs solubles de la transferrine.

Au sens de la présente invention, on entend par « fer », le fer sous la forme d'un atome de fer, de sel de fer, ou de fer organique, ou de toute formulation contenant du fer qui soit pharmaceutiquement acceptable. A titre de liste non exhaustive, le sel de fer pharmaceutiquement acceptable est sélectionné parmi les sels ferreux et les sels férriques, de préférence parmi l'ammonium citrate ferrique, le pyrophosphate ferrique, le ferrocholinate, l'ascorbate ferreux, l'aspartate ferreux, le chlorure ferreux, le sulfate ferreux, le tartrate ferreux, le fumarate ferreux, le gluconate ferreux, le gluceptate ferreux, le sulfate de glycine ferreux, le lactate ferreux, l'oxalate ferreux, le succinate ferreux. Selon un mode préféré de l'invention, le sel de fer est le sulfate ferreux, et de préférence du sulfate ferreux gastro-protégé telle la spécialité "Tardyféron" des Laboratoires Pierre Fabre Médicament. Alternativement, le fer pharmaceutiquement acceptable est sous la forme de fer dextran, de fer sucrose, de fer poly-maltose, de fer sorbitol. Lorsque le fer est sous la forme de fer organique pharmaceutiquement acceptable, il s'agit de préférence de bi-glycinate de fer, de glycinate de fer ou de fer protéine succinylate.

La nature du sel administré au patient dépend de la voie d'administration retenue qui pourra être indifféremment la voie orale, anale, parentérale, intraveineuse, intramusculaire. De préférence, il s'agit de la voie orale.

Par "symptômes du TDAH", on entend désigner notamment les troubles attentionnels tels l'inattention, l'impulsivité, l'impatience, les troubles oppositionnels, mais également l'hyperactivité motrice diurne ou nocturne, et les insomnies. Par insomnie on entend désigner :
a. l'insomnie par endormissement qui se caractérise par des difficultés à s'endormir ;
b. l'insomnie de maintenance qui se caractérise par une hyperactivité motrice nocturne et des réveils en cours de nuit, et ;
c. l'insomnie psychopathologique généralement chronique et généralement liée à une anxiété, au stress et à des épisodes dépressifs.

Selon un mode préféré de réalisation, l'utilisation du fer ou de l'un de ses sels pharmaceutiquement acceptables selon l'invention est réalisée en association avec au moins un composé sélectionné parmi les psycho-stimulants, comme produit de combinaison pour une utilisation simultanée, séparée ou échelonnée dans le temps.

Par composés psycho-stimulants, on entend désigner les inhibiteurs de la recapture de la dopamine et/ou de la noradrénaline. Parmi ceux-ci, il convient de citer à titre non exhaustif la L-Dopa, la Dopamine, les agonistes de la L-dopa, les agonistes de la dopamine. Plus particulièrement, les composés psychostimulants sont choisis parmi le methylphénidate (spécialité Ritaline®), le modafinil, l'atomoxétine, et les amphétamines, telles que la d-amphétamine, la déxédrine, la dexamphétamine.

La présente invention concerne également l'utilisation du fer, ou l'un de ses sels pharmaceutiquement acceptables en association avec au moins un composé sélectionné parmi les psycho-stimulants, notamment les inhibiteurs de la recapture de la dopamine et/ou de la noradrénaline, comme produit de combinaison pour une utilisation simultanée, séparée ou échelonnée dans le temps, pour la préparation d'un médicament destiné au traitement préventif et/ou curatif du TDAH. La composition pharmaceutique comprenant cette association et des excipients pharmaceutiquement acceptables pour une utilisation dans une methode de traitement preventif et/ou curatif du TDAH fait également partie de l'invention.

Dans le cadre de la présente invention, La posologie martiale correspond à une prise journalière de sulfate ferreux comprise entre 100 mg et 2 g par jour, et plus particulièrement, d'au moins 150 mg, d'au moins 200 mg, d'au moins 250 mg, d'au moins 300 mg, d'au moins 350 mg, d'au moins 400 mg, d'au moins 450 mg, d'au moins 500 mg, d'au moins 550 mg, d'au moins 600 mg, d'au moins 700 mg, d'au moins 800 mg, d'au moins 900 mg, d'au moins 1 g par jour, de préférence comprise entre 400 mg et 750 mg par jour, de préférence environ 500 mg, en une ou plusieurs prises quotidiennes.

Le patient selon l'invention est choisi parmi un nouveau-né, un enfant, un adolescent, un adulte. Selon un mode préféré de réalisation, il s'agit d'un enfant d'âge environ 5 à 12 ans, et/ou d'un adolescent. Le patient selon l'invention est affecté d'une carence martiale mais est non anémié, c'est-à-dire qu'il ne présente pas de baisse du taux d'hémoglobine. Par l'expression "carence martiale", on entend une hypoferritinémie sans modification significative de la concentration sérique en récepteurs solubles de la transferrine. La carence en ferritine peut être mesurée dans le sérum, mais également dans tout autres liquides biologiques tels que le liquide céphalo-rachidien.

Une carence en ferritine correspond à une concentration sérique en ferritine du patient adulte inférieure à environ 50 µg/litre. Cette hypoférritinémie peut atteindre des concentrations en ferritine inférieures à environ 40 µg/l, voire inférieures à environ 35 µg/l, inférieures à environ 30 µg/l, inférieures à environ 20 µg/l, inférieures à environ 15 µg/l, voire même inférieures à environ 10 µg/l. Les techniques de dosage de la ferritine sérique sont bien connues de l'homme de l'art. On peut citer la méthode immunoenzymatique (Kit IMX ferritine, Abott Laboratories).

Le patient selon l'invention présente en outre une concentration sérique normale de récepteurs solubles à la transferrine. La transferrine est impliquée dans l'acquisition du fer par les cellules de l'organisme ; cette acquisition est contrôlée par le nombre de récepteurs à la transferrine existant à la surface cellulaire. La concentration de ces récepteurs peut être évalués par des techniques connues de l'homme de l'art telles que la néphélémétrie (Ruivard et al., 2000 Rev. Méd. Interne 21 : 837-843). Une fourchette de concentration normale des récepteurs solubles à la transferrine est de 2,0-4,50 mg/l pour les hommes et de 1,80-4,70 mg/l pour les femmes (voir Kit RsTF Ref.2148315 de Roche).

Le rôle du fer au niveau du système nerveux central est souvent rapporté en neurophysiopathologie fondamentale comme clinique. Une asthénie fonctionnelle, intellectuelle, un syndrome de fatigue chronique, ou à l'inverse une instabilité psychomotrice et une irritabilité peuvent être la conséquence d'une carence martiale (Lozoff, 1989 Adv Pediatr 1989; 6: 331-59). Le rôle du fer dans la physiopathologie de maladies neurologiques, et notamment dans la Maladie de Parkinson Idiopathique est connu depuis plus de trente ans. L'évidence d'une augmentation martiale notamment dans certaines structures cérébrales (p.e. noyau denté) dans des pathologies neurodégénératives rares (p.e. ataxie de Friedreich) est également connue. Plus récemment, le rôle des récepteurs de la transferrine dans certains processus neurophysiopathologiques vient d'être documenté (Marder K, et al. 1998 Neurology 50, 4:1138-40). Une augmentation en nombre des récepteurs de la transferrine des cellules de l'endothélium des capillaires cérébraux pourrait être responsable de l'accumulation cytoplasmique du fer dans les cellules des neurones des ganglions de la base (globus pallidus, substantia nigra, noyau rouge, et noyau denté). Un dysfonctionnement des récepteurs de la transferrine par hyperplasie (augmentation du nombre des récepteurs) au niveau central expliquerait l'accumulation du fer dans certaines structures impliquées dans les phénomènes de neurodégénérescence. A contrario, une diminution de ces récepteurs contribuerait à protéger les noyaux centraux du phénomène. Dans l'hypothèse d'une diminution de la ferritine plasmatique dans la physiopathologie du TDAH, une augmentation physiologique des récepteurs de la transferrine devrait se produire, comme elle se produit normalement en cas d'anémie, afin de ne pas mettre les structures cérébrales en carence martiale. Par contre, une absence de réponse (absence d'augmentation du nombre des récepteurs de la transferrine) conduirait une diminution martiale cérébrale et serait compatible avec un dysfonctionnement dopaminergique par baisse de sa synthèse et/ou de la stimulation des récepteurs dopaminergiques.

Enfin l'invention vise à protéger une source de fer en association avec au moins un psychostimulant, de préférence la ritaline®, comme médicament ou comme principes actifs d'une composition pharmaceutique comprenant des excipients pharmaceutiquement acceptables, pour le traitement préventif et/ou curatif du TDAH par une posologie martiale correspondant a une prise journaliere de sulfate ferreux comprise entre 100 mg et 2 g par jour, de preference environ 500 mg, en une ou plusieurs prises.

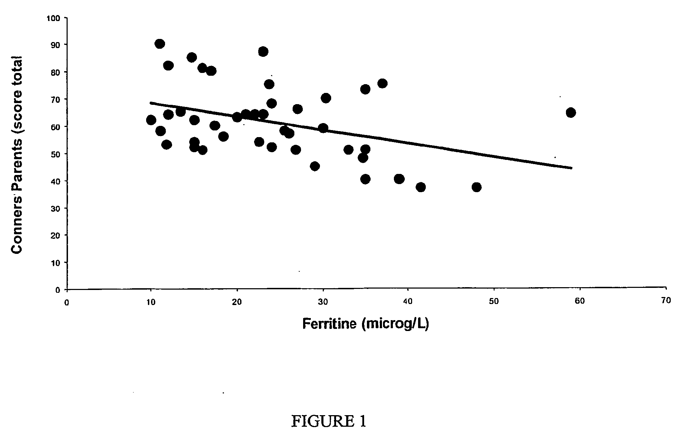

D'autres caractéristiques, buts et avantages de l'invention ressortiront des exemples qui suivent. L'invention ne se trouve pas limitée aux exemples particuliers mentionnés à simple titre illustratif et qui doivent être lus en regard de la figure suivante :
Figure 1 :
   Les valeurs de ferritine (normale = 34µg/L) sont inversement corrélées (p<0.01) à la sévérité des symptômes du TDAH exprimée par le Conners Parents (normale < 50).

### EXEMPLES

### 1. Méthode

Quarante-trois enfants, 36 garçons et 7 filles, d'un âge moyen de 9,2 ± 2,2 ans, ont participé à cette étude prospective. Leurs caractéristiques cliniques correspondaient aux critères du TDAH du Manuel diagnostique et statistique des troubles mentaux (Diagnostic and Statistical Manual of Mental Disorders) (4èmeédition, APA, 1994). Ces TDAH ont été confirmés par un entretien structuré (anamnèse). Les enfants ne souffraient d'aucune déficience physique, ni de malnutrition, ni de maladies mentales (QI>80) ou organiques et n'étaient sous aucun traitement incluant une supplémentation en fer ou des psycho-stimulants, ceci pour une durée d'au moins 2 mois avant l'étude.

La gravité des symptômes a été évaluée à l'aide de l'échelle d'évaluation de Conners (questionnaire à l'usage des parents), y compris pour les sous-échelles d'évaluation des capacités cognitives et du trouble oppositionnel. La ferritinémie a été mesurée, ainsi que le taux d'hémoglobine, l'hématocrite et le fer sérique par les méthodes classiques (Tests Elecsys, immuno-enzymologie). Les valeurs moyennes de la ferritine ont été classées en trois groupes : normal (> 34 µg/L), sub-normale (> 15 µg/L) et anormal (< 15 µg/L).

### 2. Résultats

Le résultat moyen de tous les patients (61±13) soumis au questionnaire de Conners à l'usage des parents a révélé de graves TDAH. Ce résultat a été identique chez les filles (63±8) et chez les garçons (60±14). Le fer sérique (moyenne du grouple : 84±36 µg/100 ml), les taux d'hémoglobine et d'hématocrite étaient normales. La valeur moyenne de la ferritinémie était basse (25±12 µg/L) et notamment pathologique chez 33 sur 43 enfants présentant un TDAH (77%). Elle était identique chez les garçons (26±13 µg/L) et chez les filles (19±8 µg/L) et il n'y avait pas de relation avec l'âge.

Les relations entre les caractéristiques cliniques et biologiques des patients, en fonction de leur ferritinémie (anormale, sub-normale et normale) sont reportées dans le tableau 1. Les enfants ayant une ferritine anormale présentaient des symptômes cliniques plus graves pour le TDAH que les enfants ayant une ferritine normale (p< 0.01).

D'autre part, il y avait une corrélation négative entre le questionnaire de Conners à usage des parents et les valeurs de la ferritine (r= -0,41 p= 0,01). Il n'y avait pas de corrélation entre les scores du questionnaire de Conners à usage des parents pour les symptômes d'hyperactivité, de trouble cognitif et de trouble oppositionnel, et les valeurs de la ferritine.

Tableau 1: Caractéristiques cliniques et biologiques dans les différents groupes d'enfants présentant TDAH.

| | Valeurs moyennes de la ferritine | | |
|---|---|---|---|
| Caractéristiques des patients | Basse (<15µg/L) | Sub normale (>15µg/L) | Normale (>34µg/L) |
| Nombre | 11 | 22 | 10 |
| Age | 9.1±2.5 ans | 9.6+2.1 ans | 8.7±2.2 ans |
| Sexe (M/F) | 8/3 | 18/4 | 10/0 |
| Score de Conners parental | | | |
| - Total | 66±13* | 63±11* | 52±14* |
| - Score "hyperactivité" - score "trouble | 19±4 | 19±4 | 17±4 |
| cognitif" | 10±4 | 9±4 | 8±5 |
| - score "trouble oppositionnel" | 8±3 | 9±2 | 7±2 |
| Fer sérique (µg/100 ml) | 86±27 | 81±40 | 92±44 |
| Ferritine (µg/L) | 13±2 | 23±5 | 43±11 |

Les données sont indiquées en : moyenne ± déviation standard

* p<0.015, différence statistiquement significative par rapport aux enfants avec une ferritinémie normale.

L'inventeur a mis en évidence qu'une férritinémie basse correspond à des réserves en fer basses chez les enfants souffrant de TDAH. Le point de rupture de la carence en fer chez l'enfant (entre 5 et 12 ans) est un taux de ferritine sérique supérieur à 15µg/L, et touche 3 % d'entre eux. En revanche, dans notre étude, 23% des TDAH ont révélé des carences en fer sévères sans anémie. De plus, il apparaît que les valeurs de la ferritine étaient inversement corrélées à la sévérité des symptômes exprimée par le questionnaire de Conners Parents. Ceci laisse supposer qu'il existe un rapport entre les réserves en fer (ferritine) et les symptômes du TDAH.

Il semble donc important d'instaurer le contrôle des réserves en fer de l'organisme (ferritinémie) de façon systématique en consultation spécialisée du TDAH. La recherche d'une carence martiale dans le TDAH et sa prise en charge thérapeutique devraient en effet précéder l'administration d'un psycho-stimulant.

## Revendications

1. Utilisation du fer pour la préparation d'un médicament destiné au traitement préventif et/ou curatif du trouble du déficit de l'attention/hyperactivité (TDAH), par une posologie martiale correspondant à une prise journalière de sulfate ferreux comprise entre 100mg et 2g par jour, de préférence environ 500mg, en une ou plusieurs prises.

2. Utilisation selon la revendication 1 **caractérisée en ce que** le dit patient est choisi parmi un nouveau-né, un enfant, un adolescent, un adulte.

3. Utilisation selon la revendication 2, **caractérisée en ce que** ledit patient est un enfant présentant une carence martiale isolée mais non anémié.

4. Utilisation selon l'une quelconque des revendications 1 à 3 **caractérisée en ce que** le fer est sous la forme d'un atome de fer, de sel de fer ou de fer organique, pharmaceutiquement acceptables.

5. Utilisation selon la revendication 4 **caractérisée en ce que** le sel de fer pharmaceutiquement acceptable est sélectionné parmi les sels ferreux et les sels ferriques, de préférence parmi l'ammonium citrate ferrique, le pyrophosphate ferrique, la ferritine, le ferrocholinate, l'ascorbate ferreux, l'aspartate ferreux, le chlorure ferreux, le sultate ferreux, le tartrate ferreux, le fumarate ferreux, le gluconate ferreux, le gluceptate ferreux, le sulfate de glycine ferreux, le lactate ferreux, l'oxalate ferreux, le succinate ferreux.

6. Utilisation selon la revendication 5 **caractérisé en ce que** le sel de fer est le sulfate ferreux.

7. Utilisation selon la revendication 4 **caractérisée en ce que** le fer pharmaceutiquement acceptable est sous la forme de fer dextran, de fer sucrose, de fer polymaltose, de fer sorbitol.

8. Utilisation selon la revendication 4 **caractérisée en ce que** le fer organique pharmaceutiquement acceptable est sous la forme de bi-glycinate de fer, de glycinate de fer ou de fer protéine succinylate.

9. Utilisation selon l'une quelconques des revendications précédentes **caractérisée en ce que** le médicament est formulé pour permettre l'administration du fer par voie orale, anale, parentérale, intra- musculaire ou intraveineuse.

10. Utilisation selon l'une quelconque des revendications 1 à 9 en association avec au moins un composé sélectionné parmi les psycho-stimulants, notamment les inhibiteurs de la recapture de la dopamine et/ou de la noradrénaline comme produit de combinaison pour une utilisation simultanée, séparée ou échelonnée dans le temps.

11. Utilisation selon la revendication 10 **caractérisée en ce que** le dit composé est choisi parmi le méthylphénidate, le modafinil, l'atomoxétine, et les amphétamines, notamment la d-amphétamine, la déxédrine, la dexamphétamine, la L-Dopa, la Dopamine, les agonistes de la L-dopa, et les agonistes de la dopamine.

12. Utilisation selon l'une quelconque des revendications 1 à 11 **caractérisée en ce que** le dit patient est affecté d'une carence en ferritine, la dite concentration sérique en ferritine du dit patient étant inférieure à 50 µg/l, inférieure à environ 40 µg/l, inférieure à environ 35 µg/l, inférieure à environ 30 µg/l, inférieure à environ 20 µg/l, inférieure à environ 15 µg/l, inférieure à environ 10 µg/l, inférieure à environ 5 µg/l.

13. Utilisation selon la revendication 12 **caractérisée en ce que** le dit patient présente en outre une concentration sérique normale de récepteurs solubles à la transferrine.

14. Composition pharmaceutique comprenant des excipients pharmaceutiquement acceptables, du fer ou un de ses sels pharmaceutiquement acceptables, et au moins un psychostimulant, de préférence le méthylphénidate, pour une utilisation dans une méthode de traitement préventif et/ou curatif du TDAH par une posologie martiale correspondant à une prise journalière de sulfate ferreux comprise entre 100mg et 2g par jour, de préférence environ 500mg, en une ou plusieurs prises.

## Patentansprüche

1. Verwendung von Eisen zur Herstellung eines Medikaments zur präventiven und/oder kurativen Behandlung der Aufmerksamkeitsdefizit-/Hyperaktivitätsstörung (ADHS), durch eine Eisendosierung entsprechend einer Tageseinnahme des Eisensulfats zwischen 100 mg und 2 g täglich, vorzugsweise ungefähr 500 mg, als eine oder mehrere Einnahmen.

2. Verwendung nach Anspruch 1, **dadurch gekennzeichnet, dass** der besagte Patient ausgewählt ist aus einem Neugeborenen, einem Kind, einem Jugendlichen, einem Erwachsenen.

3. Verwendung nach Anspruch 2, **dadurch gekennzeichnet, dass** der besagte Patient ein Kind ist, das einen isolierten Eisenmangel, jedoch keine Anämie aufweist.

4. Verwendung nach einem der Ansprüche 1 bis 3, **dadurch gekennzeichnet, dass** das Eisen in der Form eines pharmazeutisch annehmbaren Eisenatoms, Eisensalzes oder organischen Eisens vorliegt.

5. Verwendung nach Anspruch 4, **dadurch gekennzeichnet, dass** das pharmazeutisch annehmbare Eisensalz ausgewählt ist aus Eisen(II)salzen und Eisen(III)salzen, vorzugsweise aus Eisen(III)ammoniumcitrat, Eisen(III)pyrophosphat, Ferritin, Ferrocholinat, Eisen(II)ascorbat, Eisen(II)aspartat, Eisen(II)chlorid, Eisen(II)sulfat, Eisen(II)tartrat, Eisen(II)fumarat, Eisen(II)gluconat, Eisen(II)gluceptat, Eisen(II)glycinsulfat, Eisen(II)lactat, Eisen(II)oxalat, Eisen(II)succinat.

6. Verwendung nach Anspruch 5, **dadurch gekennzeichnet, dass** das Eisensalz das Eisen(II)sulfat ist.

7. Verwendung nach Anspruch 4, **dadurch gekennzeichnet, dass** das pharmazeutisch annehmbare Eisensalz in der Form von Eisendextran, Eisensucrose, Eisenpolymaltose, Eisensorbitol vorliegt.

8. Verwendung nach Anspruch 4, **dadurch gekennzeichnet, dass** das pharmazeutisch annehmbare organische Eisen in der Form von Eisenbiglycinat, Eisenglycinat oder Eisenproteinsuccinylat vorliegt.

9. Verwendung nach einem der vorhergehenden Ansprüche, **dadurch gekennzeichnet, dass** das Medikament so formuliert ist, dass es die Verabreichung des Eisens auf oralem, analem, parenteralem, intramuskulärem oder intravenösem Wege erlaubt.

10. Verwendung nach einem der Ansprüche 1 bis 9 in Verbindung mit mindestens einer Verbindung ausgewählt aus Psychostimulanzien, insbesondere Dopamin- und/oder Noradrenalin-Wiederaufnahme-Inhibitoren als Kombinationspräparat für eine gleichzeitige, separate oder zeitlich gestaffelte Anwendung.

11. Verwendung nach Anspruch 10, **dadurch gekennzeichnet, dass** die besagte Verbindung ausgewählt ist aus Methylphenidat, Modafinil, Atomoxetin, Amphetaminen, insbesondere d-Amphetamin, Dexedrin, Dexamphetamin, L-Dopa, Dopamin, L-Dopa-Agonisten, Dopamin-Agonisten.

12. Verwendung nach einem der Ansprüche 1 bis 11, **dadurch gekennzeichnet, dass** der besagte Patient unter einem Ferritin-Mangel leidet, wobei die besagte Ferritin-Serumkonzentration des besagten Patienten unter 50 µg/l, unter ungefähr 40 µg/l, unter ungefähr 35 µg/l, unter ungefähr 30 µg/l, unter ungefähr 20 µg/l, unter ungefähr 15 µg/l, unter ungefähr 10 µg/l, unter ungefähr 5 µg/l liegt.

13. Verwendung nach Anspruch 12, **dadurch gekennzeichnet, dass** der besagte Patient ferner eine normale Serumkonzentration an löslichen Transferrin-Rezeptoren aufweist.

14. Pharmazeutische Zusammensetzung, umfassend pharmazeutisch annehmbare Trägerstoffe, aus Eisen oder einem seiner pharmazeutisch annehmbaren Salze, und mindestens einem Psychostimulans, vorzugsweise Methylphenidat, zur Verwendung in einem Verfahren zur präventiven und/oder kurativen Behandlung von ADHS durch eine Eisendosierung entsprechend einer Tageseinnahme des Eisensulfats zwischen 100 mg und 2 g täglich, vorzugsweise ungefähr 500 mg, als eine oder mehrere Einnahmen.

## Claims

1. Use of iron for the preparation of a drug intended for the prevention and/or treatment of attention deficit hyperactivity disorder (ADHD), by an iron dosage corresponding to a daily intake of ferrous sulphate of between 100 mg and 2 g per day, preferably of about 500 mg, in one or more intakes.

2. Use as claimed in claim 1, **characterised in that** said patient is chosen from a newborn, a child, an adolescent and an adult.

3. Use as claimed in claim 2, **characterised in that** said patient is a child who exhibits an isolated iron-related deficiency but is not anaemic.

4. Use as claimed in claims 1 to 3, **characterised in that** the iron is in the form of a pharmaceutically acceptable iron atom, an iron salt or organic iron.

5. Use as claimed in claim 4, **characterised in that** the pharmaceutically acceptable iron salt is selected from ferrous salts and ferric salts, preferably from ferric ammonium citrate, ferric pyrophosphate, ferritin, ferrocholinate, ferrous ascorbate, ferrous aspartate, ferrous chloride, ferrous sulphate, ferrous tartrate, ferrous fumarate, ferrous gluconate, ferrous gluceptate, ferrous glycine sulphate, ferrous lactate, ferrous oxalate and ferrous succinate.

6. Use as claimed in claim 5, **characterised in that** the iron salt is ferrous sulphate.

7. Use as claimed in claim 4, **characterised in that** the pharmaceutically acceptable iron is in the form of iron dextran, iron sucrose, iron polymaltose or iron sorbitol.

8. Use as claimed in claim 4, **characterised in that** the pharmaceutically acceptable organic iron is in the form of iron biglycinate, iron glycinate or iron protein succinylate.

9. Use as claimed in any of the preceding claims **characterised in that** the drug is formulated so as to enable oral, anal, parenteral, intramuscular or intravenous administration.

10. Use as claimed in any of claims 1 to 9, in combination with at least one compound selected from psychostimulants, in particular dopamine and/or noradrenaline reuptake inhibitors, as a combination product for simultaneous, separate or spread out over time use.

11. Use as claimed in claim 10, **characterised in that** said compound is chosen from methylphenidate, modafinil, atomoxetine, and amphetamines, in particular d-amphetamine, dexedrine, dexamphetamine, L-Dopa, Dopamine, L-dopa agonists, and dopamine agonists.

12. Use as claimed in any one of claim 1 to 11, **characterised in that** said patient is affected by a ferritin deficiency, said serum ferritin concentration of said patient being less than 50 µg/litre, less than approximately 40 µg/l, less than approximately 35 µg/l, less than approximately 30 µg/l, less than approximately 20 µg/l, less than approximately 15 µg/l. less than approximately 10 µg/l, less than approximately 5 µg/l.

13. Use as claimed in claim 12, **characterised in that** said patient also has a normal serum concentration of soluble transferrin receptors.

14. Pharmaceutical composition comprising pharmaceutically acceptable excipients, iron or a pharmaceutically acceptable salt thereof and at least one psychostimulant, preferably methylphenidate, for a use in a method for the preventive and/or curative treatment of ADHD by an iron dosage corresponding to a daily intake of ferrous sulphate of between 100 mg and 2 g per day, preferably of about 500 mg, in one or more intakes.
